# EUROPEAN PATENT APPLICATION

(11) **EP 4 397 362 A1**
(43) Date of publication of application: **10.07.2024**
(21) Application number: 22864862.2
(22) Date of filing: 15.07.2022
(51) Int. Cl.: A61N 5/06, A61H 33/00, G01N 27/22

(54) **FLOATING ULTRAVIOLET GENERATOR**

(30) Priority: 31.08.2021 KR 20210115746
(71) Applicant: IODD Co., Ltd., Yongin-si, Gyeonggi-do 17015 (KR)
(72) Inventor: KOH, Bong Seok, Yongin-si, Gyeonggi-do 17015 (KR)
(74) Representative: Caspary, Karsten
(86) International application number: PCT/KR2022/010375
(87) International publication number: WO 2023/033353

(57) **Abstract**

A floating ultraviolet irradiator is disclosed. The floating ultraviolet irradiator according to the present invention comprises: a lower case having, at the center of a protruding part protruding downward, an opening of a predetermined area; an upper cover coupled to the lower case so as to partition a mounting space therebetween disconnected from the outside; and a substrate provided in the mounting space disconnected from the outside and having a UV light source mounted therein, and can float on water by means of air filled in the mounting space disconnected from the outside, wherein a UV transmission window is coupled to the opening of the lower case in a sealed state, a UV light source for transmitting UV light to the UV transmission window is mounted in the center of the lower side of the substrate at a position corresponding to that of the UV transmission window, and at least two water detectors for detecting contact with water are mounted along the periphery of the substrate in contact with the lower case.

## Description

### Technical Field

The present disclosure relates to an ultraviolet irradiator and, more particularly, to a floating ultraviolet irradiator that floats on water in a bathtub and emits ultraviolet rays (UV-B) in a specific wavelength range (280-320 nm), which are effective in synthesizing vitamin D in the body or treating skin diseases, toward a person taking a lower-body bath or a whole-body bath.

### Background Art

Among solar rays composed of visible rays, ultraviolet rays, and infrared rays, ultraviolet rays (UV) refers to electromagnetic radiation having a wavelength longer than X-rays and shorter than visible rays. In the electromagnetic spectrum, ultraviolet rays are close to the purple band and have a wavelength range of 100 to 3800 angstroms, which is not visible to the human eye.

Ultraviolet rays have a lower power of penetration than ordinary X-rays or gamma rays but have higher energy than visible rays, so that ultraviolet rays are known to affect human skin or small organisms. Accordingly, ultraviolet rays are generally used for sterilization and disinfection and are also called actinic rays because of strong chemical actions.

Ultraviolet rays play a beneficial role such as synthesizing vitamin D in the body and acting as a disinfectant. These ultraviolet rays are largely classified into three primary types, UV-A, UV-B, and UV-C, according to the wavelength range. Among them, UV-C, which has an excellent disinfection effect, is blocked by the ozone layer, and UV-A and UV-B affect the skin.

UV-A accounts for more than 90% of ultraviolet rays and is considered the main cause of skin aging. On the other hand, UV-B has a shorter wavelength than UV-A, and does not penetrate deep into the skin, but converts provitamin D into vitamin D, which acts to produce immune cells, and is known to be effective in treating skin diseases. However, when UV-B is directly exposed to the eyes, eye damage such as cataracts may occur, so careful attention is required.

Meanwhile, UV-C is used in various forms across a variety of industrial fields such as green algae remover, household disinfection devices, home or industrial air purifiers, and water treatment devices due to its excellent sterilization effect. On the other hand, despite its effectiveness in synthesizing vitamin D in the body and treating skin diseases as mentioned above, UV-B is not widely utilized due to problems of conventional technologies.

An example of a conventional technology that uses UV-B to synthesize vitamin D in the body or treat skin diseases is a stand-up type of a skincare device. In order to use such a product that emits ultraviolet rays by attaching an ultraviolet lamp to the upper end of the stand, it is necessary to wear dedicated goggles (goggles for UV protection) for safety and it is cumbersome to change clothes for use since the body should be exposed.

That is, the conventionally known stand-type of a skincare device has problems in that dedicated goggles should be worn for use, there is the inconvenience of having to change separate clothes to expose the skin, and there is also the inconvenience of storage and usage due to the stand for fixing the ultraviolet lamp at a certain height.

### [Document of Related Art]

(Patent Document 1) Korean Patent Application Publication No. 10-2018-0107074 (Publication Date: October 1, 2018)

### Disclosure

### Technical Problem

A technical task that the present disclosure seeks to solve is to provide a floating ultraviolet irradiator that helps synthesize vitamin D in the body or treat skin diseases by emitting toward the body in the water the ultraviolet rays (UV-B) in a specific wavelength range (280-320 nm) while floating on the water filled in the bathtub when a person takes a lower-body bath or whole-body bath.

Another technical task that the present disclosure seeks to solve is to provide a floating ultraviolet irradiator that promotes safe use by blocking ultraviolet rays (UV-B) from being directly exposed to a user's eyes through automatically detecting when the device is lifted or is heaving or overturned due to waves while floating on water and immediately stopping the generation of ultraviolet rays (UV-B) .

Another technical task that the present disclosure seeks to solve is to provide a floating ultraviolet irradiator that reduces the inconvenience caused by changing clothes, does not require dedicated goggles thanks to added safety functions (a function that automatically detects when heaving or flipping over occurs and immediately stops the generation of ultraviolet rays (UV-B)), and increases the convenience of use by making it easy to store.

### Technical Solution

According to the present disclosure as a means of solving the problems, provided may be a floating ultraviolet irradiator that includes
a lower case having an opening of a predetermined area in the center,
an upper cover to be coupled to the lower case in order to partition a mounting space disconnected from the outside between the lower case and thereof, and
a substrate which is installed in the mounting space disconnected from the outside and on which a UV light source is mounted,
wherein the irradiator is capable of floating on the water by a buoyancy generating means in the mounting space disconnected from the outside,
a UV transmission window is coupled to the opening of the lower case to be in a sealed state,
a UV light source to emit UV rays is mounted on the center of a lower surface of the substrate at a position corresponding to that of the UV transmission window, and
at least more than two water detectors that detect contact with water are mounted around the periphery of the substrate in contact with the lower case.

In addition, the present disclosure may include a main controller mounted on an upper surface of the substrate opposite to the surface where the UV light source is mounted and the main controller is provided with a gravity sensor (G Sensor).

In this case, the main controller may be driven to immediately stop the generation of UV rays by cutting off the power supply to the UV light source when some of at least more than two water detectors do not detect contact with water in the switch ON state.

In addition, the main controller may be driven to immediately stop the generation of UV rays by cutting off the power supply to the UV light source when the output of the gravity sensor exceeds a range of a preset normal output in the switch ON state.

In addition, the present disclosure further may include a plurality of body detection sensors electrically connected to the main controller and mounted on the peripheral surface of the upper cover, and in this case, the main controller may immediately stop the generation of UV rays by cutting off the power supply to the UV light source when the body is not detected within a preset detection range of the body detection sensors in the switch ON state.

In addition, the present disclosure may include a user detection pad installed in the water and composed of a load cell and a wireless transmitter for transmitting information, wherein a wireless receiver mounted on the substrate is configured to receive a signal transmitted by the user detection pad.

In this case, the main controller may be driven to immediately stop the generation of UV rays by cutting off the power supply to the UV light source when a user load is not detected on the user detection pad in the switch ON state.

In addition, a timer function may be added to the main controller applied to the present disclosure in order to stop the generation of UV rays by cutting off the power supply to the UV light source when a preset time has elapsed.

In addition, in the floating ultraviolet irradiator according to the present disclosure, a light-blocking visor that blocks UV rays reflected in the water from returning to the surface of the water may be formed continuously in the peripheral direction on the radially outermost surface of the upper cover or lower case.

Preferably, the water detector may be a capacitance sensor or a resistance measuring sensor that measures the electrical resistance of water, and at least more than two detectors may be disposed at equal intervals around the periphery of the substrate.

In addition, the buoyancy generating means applied to the floating ultraviolet irradiator according to the present disclosure may be air being filled in the mounting space disconnected from the outside or a float made of a material having a specific gravity less than 1, such as Styrofoam.

In addition, the floating ultraviolet irradiator according to the present disclosure may include a safety distance maintaining member to be coupled to the lower case in order to maintain a minimum safety distance from the user's body located in the water when used while floating on water.

### Advantageous Effects

According to the present disclosure, the floating ultraviolet irradiator may float on the water by its own buoyancy and is configured to irradiate ultraviolet rays (UV-B) in a specific wavelength range (280 ~ 320 nm) toward the water while floating in a normal posture on the water. Accordingly, the user may have the effects of synthesizing vitamin D in the body or treating skin diseases by using the present disclosure when taking the lower body bath or the whole body bath.

In addition, while floating on the water, the present disclosure may automatically detect safety-threatening events when the user lifts arbitrarily the device or the device is heaving or overturned due to waves, and then immediately stop the generation of UV rays, thereby preventing safety accidents according to direct exposure of ultraviolet rays (UV-B) to the user's eyes and consequently improving the reliability of safety.

In addition, the present disclosure may be used while floating on the water when taking a lower-body bath or a whole-body bath, and has the advantages of reducing the inconvenience caused by changing clothes, not requiring dedicated goggles thanks to added safety functions (a function that automatically detects when heaving or flipping over occurs and immediately stops the generation of ultraviolet rays (UV-B)), and increasing the convenience of use by making it easy to store.

### Description of Drawings

FIG. 1 is an exploded perspective view of a floating ultraviolet irradiator according to an exemplary embodiment of the present disclosure.
FIG. 2 is a combined perspective view of a floating ultraviolet irradiator shown in FIG. 1.
FIG. 3a is a cross-sectional view of a floating ultraviolet irradiator when viewed in a direction from the line A-A shown in FIG. 2.
FIG. 3b is a cross-sectional view showing a floating ultraviolet irradiator when a resistance measuring sensor which is a method of being in direct contact with water is applied as a water detector.
FIG. 4 is a bottom perspective view of a substrate shown in FIG. 1.
FIG. 5 is a schematic diagram of a floating ultraviolet irradiator according to an exemplary embodiment of the present disclosure.
FIG. 6 is an exemplary diagram of a use state showing the use state of a floating ultraviolet irradiator according to an exemplary embodiment of the present disclosure.
FIG. 7 is a diagram showing another preferred exemplary embodiment of a floating ultraviolet irradiator according to the present disclosure.
FIG. 8 is a diagram showing another preferred exemplary embodiment of a floating ultraviolet irradiator according to the present disclosure.
FIG. 9 is a diagram showing another preferred exemplary embodiment of a floating ultraviolet irradiator according to the present disclosure.
FIG. 10 is a diagram showing another preferred exemplary embodiment of a floating ultraviolet irradiator according to the present disclosure.

### Mode for Invention

Hereinafter, preferred exemplary embodiments of the present disclosure will be described in detail with reference to the accompanying drawings.

The terms used in the specification are used only to describe specific exemplary embodiments and are not intended to limit the present disclosure. Singular expressions include plural expressions unless the context clearly means otherwise.

Terms such as "include" or "have" in this specification are intended to designate the existence of features, numbers, steps, operations, components, parts, or a combination thereof described in the specification, and should be understood not to preclude the possibility of the existence or addition of one or more other features or numbers, steps, operations, components, parts, or combinations thereof.

In addition, terms such as first, second, and the like may be used to describe various components, but the components should not be limited by the terms. The terms are only used to distinguish one component from another component.

In addition, terms such as "... part", "... unit", and "... module" described in the specification refer to a unit that processes at least one function or operation, which may be implemented by hardware or software or a combination of hardware and software.

In the description with reference to the accompanying drawings, the same reference numerals are assigned to the same components, and overlapping descriptions thereof will be omitted. And in describing the present disclosure, when it is determined that the detailed description of the related known technology may unnecessarily obscure the gist of the present disclosure, the detailed description thereof will be omitted.

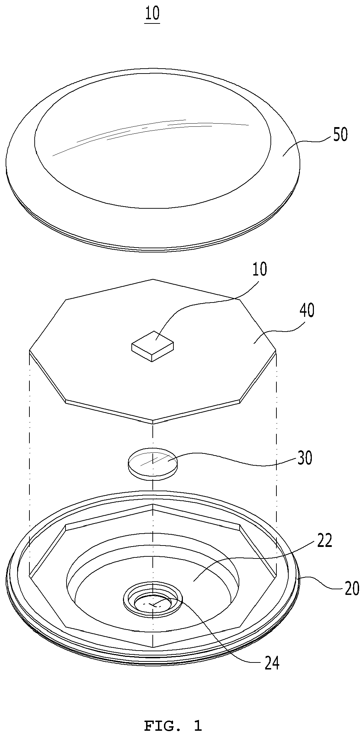

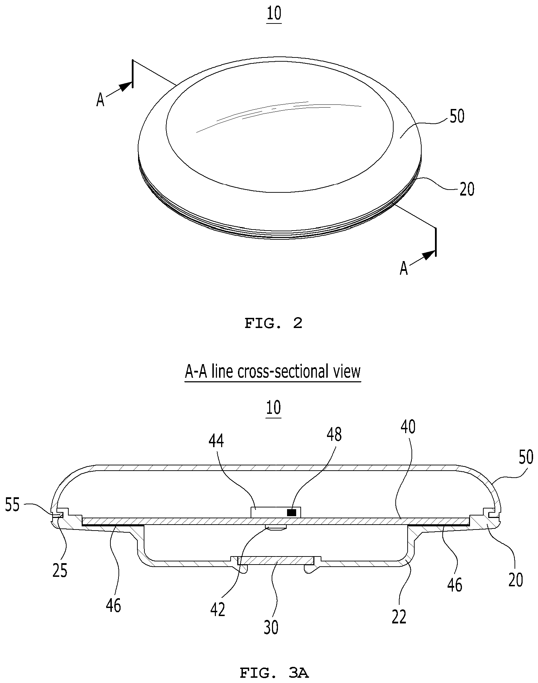

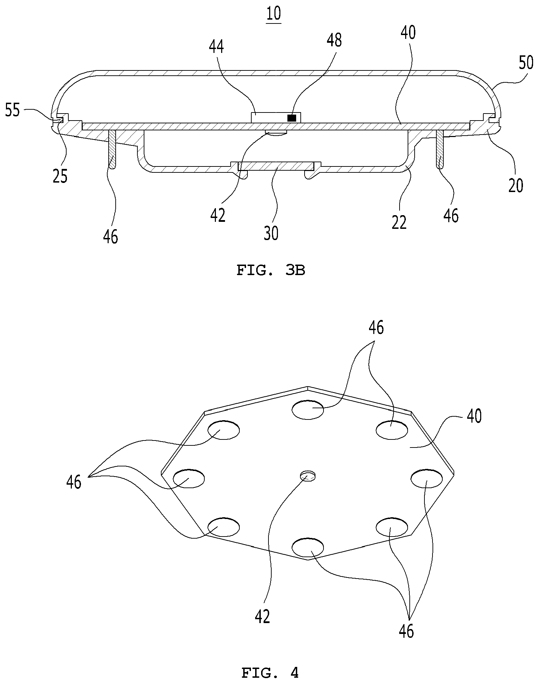

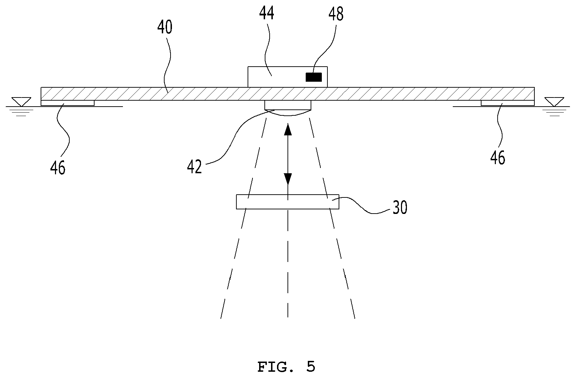

FIG. 1 is an exploded perspective view of a floating ultraviolet irradiator according to an exemplary embodiment of the present disclosure. FIG. 2 is a combined perspective view of a floating ultraviolet irradiator shown in FIG. 1. FIG. 3a is a cross-sectional view of a floating ultraviolet irradiator when viewed in a direction from the line A-A shown in FIG. 2, FIG. 4 is a bottom perspective view of a substrate shown in FIG. 1., and FIG. 5 is a conceptual diagram that extracts and shows only the main components of the present disclosure.

Referring to FIGS. 1 to 5, a floating ultraviolet irradiator 10 according to an exemplary embodiment of the present disclosure may largely include a lower case 20, an upper cover 50 to be coupled thereto, and a substrate 40 installed in a mounting space (an interior space disconnected from the outside) that is formed by combining the lower case 20 and the upper cover 50. Herein, the lower case 20, the upper cover 50, and the substrate 40 may be shown in an octagonal shape in the drawings, but are not limited thereto.

As shown in the drawing, the lower case 20 may be in the form of a polygonal (or circular) container having a protruding part 22 protruding downward to the extent of a specific height while the upper part where the upper cover 50 is coupled is open. The lower case 20 may be composed of an insulator such as plastic, and an opening 24 of a predetermined area may be formed in the center of a protruding part 22 protruding downward. In addition, the UV transmission window 30 may be coupled to the opening 24 to be in a sealed state.

As shown in the drawing, when the protruding part 22 is formed to protrude downward to the extent of a specific height in the lower case 20 and the water detector 46 described later is disposed at a position higher than the protruding part 22, the water detector 46 may detect even small waves by being spaced apart from the water surface, thereby increasing operating sensitivity and promoting safer use, compared to the configuration (not shown) in which the bottom of the lower case is entirely flat and the water detector is disposed approximately in the same line as the transmission window.

The UV transmission window 30 may be composed of transparent or translucent synthetic resin or glass that may transmit ultraviolet rays (UV-B) generated by a UV light source 42 described later. Additionally, the UV light source 42 that transmits UV rays through the UV transmission window 30 may be mounted on one surface of the substrate 40. Preferably, the UV light source 42 may be mounted in the center of the lower surface of the substrate 40 at a position corresponding to that of the UV transmission window 30 on the basis of the height direction of the product as shown in FIGS. 3a and 5.

Preferably, the UV light source 42 may be an ultraviolet light emitting diode (UV LED) that generates UV-B with a light wavelength range of 280 to 320 nm and the main controller 44 may be mounted on the other surface (the upper surface of the substrate 40) opposite to the surface on which the UV light source 42 is mounted. At this time, the main controller 44 may control the power supply between the built-in battery (not shown) and the UV light source 42 on the basis of the output of the water detector 46 and the gravity sensor 48 to be described later.

Contrary to the lower case 20, the upper cover 50 may be configured to be in the form of a polygonal (or circular) container with a lower part open. Like the lower case 20, the upper cover 50 may be composed of an insulator such as plastic, and may be tightly coupled to the lower case 20 at the upper part of the lower case 20 to partition a mounting space disconnected from the outside, thereby preventing the internal substrate 40 from moisture penetration therebetween.

For example, the combination of the upper cover 50 and the lower case 20 may be implemented (when the planar shape of the upper cover 50 and lower case 20 is polygonal) to be tightly coupled to each other using a protrusion-groove (or groove-protrusion) coupling method by configuring each of a protrusion and a groove (55 and 25, or grooves and protrusions) that may be mutually interlocked with each other in the peripheral direction at the inner surface of the open end of the upper cover 50 and the outer surface of the open end of the lower case 20 as shown in the drawing (a cross-sectional view in FIGS. 3a and 3b).

Although not shown, the upper cover 50 and the lower case 20 may be tightly coupled to each other through screw coupling between the female screw threads and the male screw threads by configuring a female screw thread to be formed on the inner surface of the open end of the upper cover 50 and a male screw thread to be formed on the outer surface of the open end of the lower case 20. This coupling method may be a coupling method applicable when the planar shape of the upper cover 50 and the lower case 20 is a circular planar shape.

A sealed mounting space disconnected from the outside may be formed inside as described above due to the tight coupling of the upper cover 50 and the lower case 20 as mentioned above. At this time, the ultraviolet irradiator according to an exemplary embodiment of the present disclosure may float on the water by the buoyancy generating means in the sealed mounting space and a water detector 46 may be mounted on the periphery of the substrate 40 in contact with the edge portion of the lower case 20.

Herein, the buoyancy generating means applied to the floating ultraviolet irradiator according to the present disclosure may be air being filled in the mounting space disconnected from the outside or a float made of a material having a specific gravity less than 1, such as Styrofoam.

The water detector 46 may serve to detect contact with water when the ultraviolet irradiator according to the exemplary embodiment of the present disclosure floats on the water with its own buoyancy (the buoyancy due to air filled in the sealed mounting space). The water detector 46 may preferably be a capacitance sensor that detects contact with water from a capacitance that changes upon contact with water and may be mounted in a plurality of two or more around the periphery of the substrate 40.

The water detector 46 may be not limited to the capacitance sensor. In some cases, a resistance measuring sensor that measures electrical resistance by being in direct contact with water may be applied as the water detector. In this case, as shown in FIG. 3b, each of the water detectors 46 may be configured to pass through a sensor penetration hole (not shown) formed at a corresponding position of the lower case 20 such that a portion of the lower end protrudes downward from the lower case 20.

At least more than two water detectors 46 may be disposed at equal intervals in the peripheral direction on the edge (a portion that contacts the edge of the lower case 20 when assembling the product) of the lower surface of the substrate 40. For example, in the case of eight as shown in FIG. 4, the detectors may be mounted on the edge of the lower surface of the substrate 40 at 45 degree intervals and the information (whether or not it is in contact with water) sensed by the water detector 46 may be provided to the main controller 44.

The main controller 44 may control the power supply to the UV light source 42 on the basis of information provided by the water detector 46. More specifically, the ultraviolet irradiator 10 according to an exemplary embodiment of the present disclosure may immediately stop the generation of UV rays by cutting off the power supply to the UV light source 42 when some of a plurality of water detectors 46 do not detect contact with water in the switch ON state.

In other words, the main controller 44 may normally allow the power supply to the UV light source 42 when the ultraviolet irradiator 10 according to the present disclosure is so stably floating on the water that all water detectors 46 are in contact with water and may immediately cut off the power supply and prevent UV rays from being directly exposed to a user's eyes when the device is lifted or is heaving or overturned due to contact with a user or waves while floating on water.

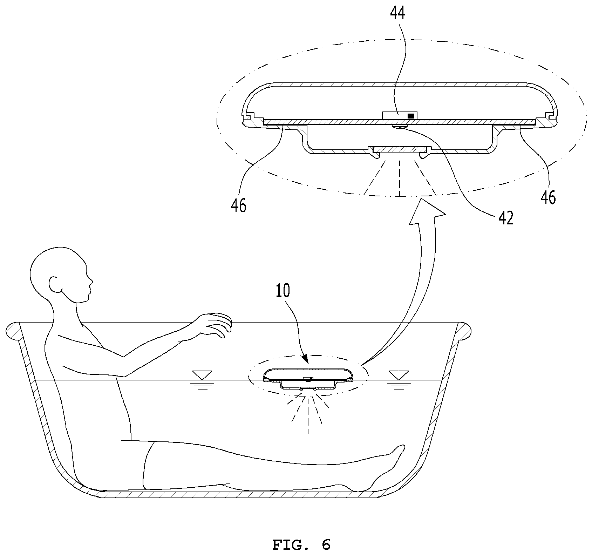

More details will be described with reference to the operation state diagram of FIG. 6.

FIG. 6 is a view showing a use state of a floating ultraviolet irradiator according to an exemplary embodiment of the present disclosure, and is an exemplary view of a use state of the present disclosure when taking a lower-body bath or a whole-body bath in a state in which the ultraviolet irradiator according to an exemplary embodiment of the present disclosure is floating on water filled in a bathtub.

As shown in the example of the use state of FIG. 6, the floating ultraviolet irradiator 10 according to an exemplary embodiment of the present disclosure may be used while floating on water filled in a bathtub. More specifically, when all of the plurality of water detectors 46 detect contact with water in a state that a user turns on a switch (not shown) and floats the irradiator, the UV light source 42 may be emitted under the control of the main controller 44.

In other words, not only when switched on, but also when all water detectors 46 detect contact with water, the main controller 44 may determine that the ultraviolet irradiator 10 according to the exemplary embodiment of the present disclosure is stably floating on the water and may allow power to be supplied to the UV light source 42, and then when the UV light source 42 enables to emit light by the allowed power supply, ultraviolet rays (UV-B) are emitted toward the body in the water, thereby synthesizing vitamin D in the body or being effective in treating skin diseases.

Meanwhile, when ultraviolet rays (UV-B) are directly exposed to the user's eyes, eye damage such as cataracts may occur, so careful attention is required. Accordingly, the present disclosure is configured to prevent ultraviolet rays from being exposed to the user's eyes by immediately cutting off the power supply to the UV light source 42 when some of the water detectors 46 do not detect contact with water because the device (10, referring to 'the ultraviolet irradiator') is lifted or overturned due to user's contact or waves while floating on the water.

In this way, the present disclosure may promote safe use by preventing in advance the user's eyes from being exposed to ultraviolet rays as a result of enabling the UV light source 42 to operate normally when the device 10 is stably floating on the water (in a state all water detectors 46 detect contact with water) and cutting off the power supply at the moment when any one of the water detectors 46 falls from the water surface when the device is heaving or overturned due to waves caused by a user's movements.

The floating ultraviolet irradiator 10 according to an exemplary embodiment of the present disclosure may include a gravity sensor (G sensor) 48 as an additional safety device in addition to the water detector 46 (see FIGS. 3a and 3b) . The gravity sensor 48 may detect a change in the direction of gravity acting on the device and may provide the information to the main controller 44 in the form of a voltage signal. The gravity sensor 48 may be provided in a module form in the main controller 44 described above or may be separately mounted on the substrate 40.

The main controller 44 may receive the detection information of the gravity sensor 48 and may determine whether the direction of gravity changes. More specifically, when the device 10 is in the switch ON state and the output (voltage signals) of the gravity sensor 48 exceeds a range of a preset normal output, it is determined that the device is tilted or overturned due to waves caused by a user's movements and the power supply to the UV light source 42 may be immediately cut off to stop the generation of UV rays.

In this way, when the gravity sensor 48 is added as a safety device together with the water detector 46, the main controller 44 may be equipped with a processor that is programmed to immediately stop the generation of UV rays when some of the plurality of water detectors 46 do not detect contact with water or when the output of the gravity sensor 48 detecting a change in the direction of gravity exceeds the preset range in a state when the device 10 floats on the water surface and operates normally.

That is, when the water detector 46 and the gravity sensor 48 are applied together as a safety device, the main controller 44 may determine that the device 10 is normally floating on the water only under the conditions that all water detectors 46 detect contact with water and the output of the gravity sensor 48 is in the normal range and then allow the generation of UV rays, and may determine that other conditions are conditions that affect the user's safety and then immediately stop the generation of UV rays.

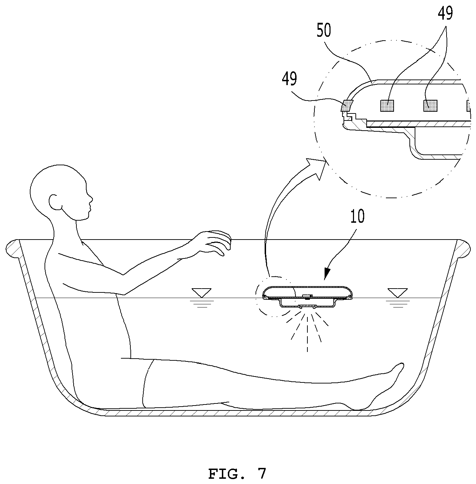

FIG. 7 is an exemplary diagram of the use state showing the use state of a floating ultraviolet irradiator according to another preferred exemplary embodiment of the present disclosure, and shows a configuration in which a body detection sensor is further added in addition to the configuration of the exemplary embodiment described above.

As shown in FIG. 7, the floating ultraviolet irradiator 10 according to the present disclosure may further include a body detection sensor 49. The body detection sensor 49 may be mounted at regular intervals in the peripheral direction of the upper cover 50 to be electrically connected to the main controller 44 on the upper surface of the substrate 40 and serve to detect whether the body is exposed above the surface of the water within a preset sensing range in a state when the device 10 is floating in a normal position on the surface of water, that is, floating on the water, and transmit the corresponding information to the main controller 44.

The fact that no person is detected within the preset sensing range in a state when the device 10 is floating in a normal posture on the water filled in the bathtub may mean in another way that there is no person in the bathtub or that a person is completely submerged under the water in the bathtub (a situation where a small child submerges in the water for fun), so in this case (when the device is floating on the water in a normal posture and the body is not detected) the main controller 44 may be set to stop the generation of UV rays for power saving and user safety.

In some cases, a timer function may be added to the main controller 44 for use for a longer time through power saving. The timer function may be a function for automatically cutting off the power supply to the UV light source 42 and stopping the generation of UV rays when a set time has elapsed while the ultraviolet irradiator according to an exemplary embodiment of the present disclosure is operating normally, and may be set through an input device (not shown) separately provided on the upper cover 50.

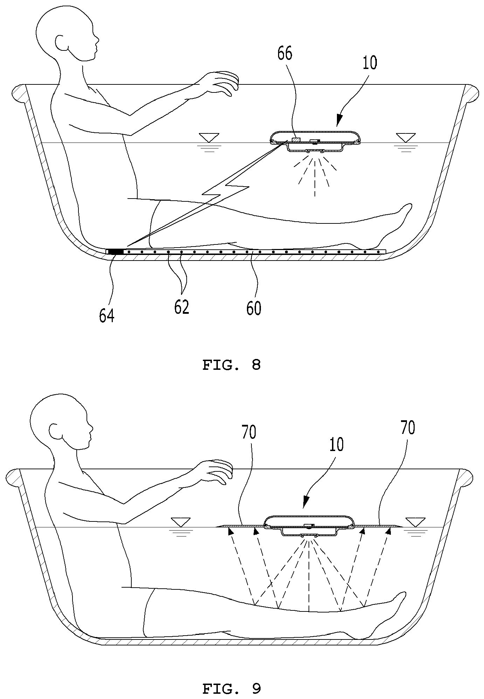

FIG. 8 is an exemplary diagram showing the use state of a floating ultraviolet irradiator according to another preferred exemplary embodiment of the present disclosure, and is an exemplary embodiment to promote power saving and user safety by placing a user detection pad 60 in the water on the bottom of a bathtub, unlike the body detection sensor described above.

Referring to FIG. 8, in addition to the floating ultraviolet irradiator 10 in the form of a single object according to the exemplary embodiment described above, a floating ultraviolet irradiator according to another preferred exemplary embodiment of the present disclosure may include a user detection pad 60 that includes a wireless transmitter 64 for transmitting information and preferably a load cell 62, a load detection element which is installed on the bottom of a bathtub in the water and detects a user load.

A wireless receiver 66 may be mounted on the substrate 40 to wirelessly receive a signal transmitted by the user detection pad 60, and a processor programmed to stop the generation of UV rays by cutting off the power supply to the UV light source 42 may be mounted on the main controller 44 when the information that a user load is not detected is received from the user detection pad 60 in a state when the device 10 is floating in a normal posture on the water after the switch is on.

Another exemplary embodiment of the present disclosure with such a configuration may normally emit UV rays when the device 10 is floating in a normal posture on the water in the switch ON state and a user load is detected from the user detection pad 60, and may stop the operation for power saving and safety when it is determined that there is no user when the device is floating in a normal posture on the water but the user housing is not detected.

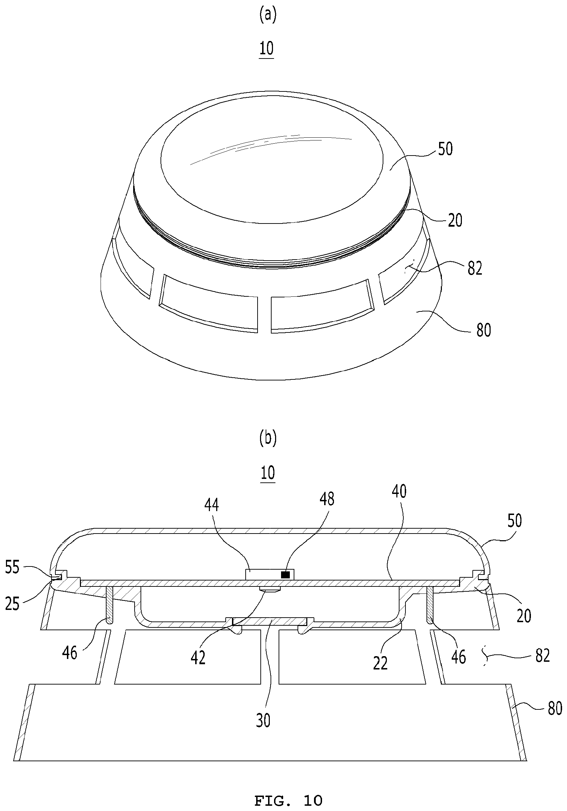

Meanwhile, FIGS. 9, 10a and 10b are views showing another preferred exemplary embodiment of a floating ultraviolet irradiator according to the present disclosure.

First, FIG. 9 is a modified example of adding a light-blocking visor 70 continuously in the peripheral direction on the radially outermost surface of the upper cover 50 or lower case 20 and is an exemplary embodiment of reliably blocking through the light-blocking visor 70 the situation where the UV rays are exposed to the user's eyes at the moment when the emitted UV rays while floating on water is reflected on the surface of the bathtub and returned to the surface of the water.

Additionally, FIGS. 10a and 10b are an exemplary embodiment of adding a safety distance maintaining member 80 that maintains a minimum safety distance so that the body of the user located in the water is not too close to the UV light source 42 while floating on the water when using the floating UV irradiator according to the present disclosure. Herein, the safety distance maintaining member 80 may be configured to be coupled to the lower case 20 and be extended downward from the lower case 20 to be located in the water.

Preferably, the safety distance maintaining member 80 may be in the shape of a skirt or a barrel in which a plurality of water passage holes 82 are formed in the peripheral direction as shown in the drawing (FIGS. 10a and 10b), but is not limited thereto. The safety distance maintaining member may be of any shape as long as it is a structure that induces the heaving of the device while being located under the water by touching first the user's body before the user's body in the water directly touches the lower case 20.

Various exemplary embodiments of the floating ultraviolet irradiator according to the present disclosure discussed above may be configured to float on the water and to emit ultraviolet (UV-B) in a specific wavelength range (280 to 320 nm) toward the water in a state floating on the water. Accordingly, the user may have the effects of synthesizing vitamin D in the body or treating skin diseases by using the present disclosure when taking a lower-body bath or a whole-body bath.

In addition, while floating on the water, the present disclosure may automatically detect when the user lifts arbitrarily the device or the device is heaving or overturned due to waves, and then immediately stop the generation of UV rays, thereby preventing safety accidents according to direct exposure of ultraviolet rays (UV-B) to the user's eyes and consequently improving the reliability of safety.

In the above-detailed description of the present disclosure, only particular exemplary embodiments have been described. However, the present disclosure should be understood as being not limited to the particular exemplary embodiments mentioned in the detailed description, but rather as including all variations, equivalents and substitutes within the spirit and scope of the present disclosure defined by the accompanying claims.

### Description of Reference Numerals

- 10:: ultraviolet irradiator
- 20:: lower case
- 22:: protruding part
- 24:: opening
- 30 :: UV transmission window
- 40 :: substrate
- 42 :: UV light source
- 44 :: main controller
- 46 :: water detector
- 48 :: gravity sensor
- 49 :: body detection sensor
- 50 :: upper cover
- 60 :: user detection pad
- 62 :: load cell
- 64 :: wireless transmitter
- 66 :: wireless receiver
- 70 :: light-blocking visor
- 80 :: safety distance maintaining member

## Claims

1. A floating ultraviolet irradiator, the irradiator comprising:
a lower case having an opening of a predetermined area in the center;
an upper cover to be coupled to the lower case in order to partition a mounting space disconnected from an outside between the lower case and thereof; and
a substrate which is installed in the mounting space disconnected from the outside and on which a UV light source is mounted,
wherein the irradiator is capable of floating on the water by a buoyancy generating means in the mounting space disconnected from the outside,
a UV transmission window spaced a predetermined distance away from the UV light source is coupled to the opening of the lower case to be in a sealed state,
the UV light source to emit UV rays toward the UV transmission window is mounted on the center of a lower surface of the substrate at a position corresponding to that of the UV transmission window, and
at least more than two water detectors that detect contact with water are mounted on the periphery of the substrate in a contact with the lower case.

2. The irradiator of claim 1, further comprising a main controller mounted on an upper surface of the substrate opposite to a surface where the UV light source is mounted, wherein the main controller is provided with a gravity sensor (G Sensor).

3. The irradiator of claim 2, wherein the main controller is driven to immediately stop an generation of UV rays by cutting off a power supply to the UV light source when some of at least two water detectors do not detect the contact with water in a switch ON state.

4. The irradiator of claim 2, wherein the main controller is driven to immediately stop the generation of UV rays by cutting off the power supply to the UV light source when an output of the gravity sensor exceeds a range of a preset normal output in the switch ON state.

5. The irradiator of claim 2, further comprising a user detection pad which is installed in the water and comprises a load cell and a wireless transmitter for transmitting information, wherein a wireless receiver mounted on the substrate is configured to receive a signal transmitted by the user detection pad.

6. The irradiator of claim 5, wherein the main controller is driven to immediately stop the generation of UV rays by cutting off the power supply to the UV light source when a user load is not detected on the user detection pad in the switch ON state.

7. The irradiator of claim 2, wherein a timer function is added to the main controller in order to stop the generation of UV rays by cutting off the power supply to the UV light source when a preset time has elapsed.

8. The irradiator of claim 1, wherein the water detector is a capacitance sensor or a resistance measuring sensor that measures the electrical resistance of water, and at least more than two detectors are disposed at equal intervals on the periphery of the substrate.

9. The irradiator of claim 1, wherein the buoyancy generating means is an air being filled in the mounting space disconnected from the outside or a float made of a material having a specific gravity less than 1.

10. The irradiator of claim 1, further comprising a safety distance maintaining member to be coupled to the lower case in order to maintain a minimum safety distance from a user's body located in the water when used while floating on the water.
